# EUROPEAN PATENT SPECIFICATION

(11) **EP 4 191 223 B1**
(45) Date of publication and mention of the grant of the patent: **11.09.2024**
(21) Application number: 21212451.5
(22) Date of filing: 06.12.2021
(51) Int. Cl.: G01N 1/04, E21C 51/00, G01N 33/24, G01N 1/08

(54) **REGOLITH SAMPLING APPARATUS AND ARM**
REGOLITH-ABTASTGERÄT UND ARM
APPAREIL ET BRAS D'ÉCHANTILLONNAGE DU RÉGOLITHE

(43) Date of publication of application: 07.06.2023
(73) Proprietor: The Hong Kong Polytechnic University, Kowloon (HK)
(72) Inventor: YUNG, Kai Leung, Hong Kong (HK); KO, Sui Man, Hong Kong (HK); KWAN, Folk Year, Hong Kong (HK); JIANG, Shui Qing, Hong Kong (HK); DU, Xiao Dong, Hong Kong (HK)
(74) Representative: Purdylucey Intellectual Property

(56) References cited:
- ANONYMOUS: "PolyU-developed space instruments complete lunar sampling for Chang'e 5 | The Hong Kong Polytechnic University", 8 December 2020 (2020-12-08), XP055920352, Retrieved from the Internet <URL:https://www.polyu.edu.hk/en/media/media-releases/2020/1208_polyu_space_instruments_complete_lunar_sampling_for_chang_e-5/> [retrieved on 20220511]
- THE HONG KONG POLYTECHNIC UNIVERSITY: "Space Exploration - PolyU's Surface Sampling and Packing System", 29 December 2020 (2020-12-29), XP055920348, Retrieved from the Internet <URL:https://www.youtube.com/watch?v=D1uNKNuYjfc> [retrieved on 20220511]
- ANONYMOUS: "PolyU develops space instruments for the Nation's first lunar sample return mission | The Hong Kong Polytechnic University", 24 November 2020 (2020-11-24), XP055920349, Retrieved from the Internet <URL:https://www.polyu.edu.hk/en/media/media-releases/2020/1124_polyu-develops-space-instruments-for-the-nations-first-lunar-sample-return-mission/> [retrieved on 20220511]
- ANONYMOUS: "PolyU-developed space instruments complete lunar sampling for Chang'e 5 | Mainland Development Office", 22 February 2021 (2021-02-22), XP055920351, Retrieved from the Internet <URL:https://www.polyu.edu.hk/mdo/news-and-events/2020/20201208/> [retrieved on 20220511]
- ??: "Hong Kong researchers help complete lunar sampling for Chang'e-5 - Chinadaily.com.cn", 18 December 2020 (2020-12-18), XP055920353, Retrieved from the Internet <URL:https://www.chinadaily.com.cn/a/202012/18/WS5fdc1710a31024ad0ba9c9ef_1.html> [retrieved on 20220511]

## Description

### FIELD OF THE DISCLOSURE

The present disclosure relates to a remotely operable sampling apparatus and arm for sampling regolith in a non-terrestrial environment, particularly suitable for sampling non-terrestrial regolith.

### BACKGROUND OF THE DISCLOSURE

The analysis of extra-terrestrial regolith samples provides unique scientific insights into the properties, formation and change in surface environment over time of the various bodies and have transformed understanding of our solar system. (As is known in the art, regolith is the unconsolidated loose heterogeneous superficial deposits covering solid rock).

Various approaches have been employed in sample collection especially in obtaining samples from Earth's nearest neighbour, the Moon. These approaches range from human facilitated retrieval (e.g. Apollo programme retrieving regolith samples from the moon between 1969 and 1972) to robotic sampling of regolith (e.g. Soviet Luna missions obtaining a hollow tube filled with soil in 1976).

Necessarily, after withstanding vibrations and other mechanical shocks associated with launch, cruising and landing, robotic sampling apparatus must be able to operate reliably under extreme conditions in various modes to collect and transfer samples. In particular, it would be appreciated jamming of the sampling apparatus on a regolith sample in the middle of the sampling process must be avoided as it could potentially jeopardise the sampling mission. At the same time any sampling apparatus must be configured to minimize weight and volume it occupies in the spacecraft. "PolyU-developed space instruments complete lunar sampling for Chang'e 5 | The Hong Kong Polytechnic University", 8 December 2020, describes how a research team at The Hong Kong Polytechnic University developed a system which accomplished the tasks of automatic sample collection and packaging on the lunar surface.

These constraints mean that the development of remotely operable sampling apparatus is uniquely challenging, and the present disclosure teaches a sampling assembly which is equipped to address at least some of the deficiencies of prior techniques and provide a useful choice.

### SUMMARY OF THE DISCLOSURE

Features and advantages of the disclosure will be set forth in the description which follows, and in part will be obvious from the description, or can be learned by practice of the herein disclosed principles. The features and advantages of the disclosure can be realized and obtained by means of the instruments and combinations particularly pointed out in the appended claims.

In accordance with a first aspect of the present disclosure, there is provided a regolith sampling apparatus. The regolith sampling apparatus comprises: a generally cylindrical outer housing extending about a longitudinal axis comprising upper, intermediate and lower housings; an inner housing received in said outer lower housing and comprising a circumferential wall defining a void therein, said inner housing being rotatable with the outer housing; a plurality of retractable flaps extending from between the inner housing and lower outer housing; a piston member comprising a shaft and a piston head disposed in the inner housing; a flap support member translatable along on a first threaded rod upon rotation thereof, said flap support member being coupled to said retractable flaps to urge said flaps into and out from the space between the inner housing and the lower housing upon translation of said flap support member on said threaded rod; an upper platform rotatably isolated and supported in the upper outer housing at a fixed height therein, said upper platform supporting a flap driving means rotatably coupled to the first threaded rod via a first gear train and a piston member driving means coupled to the outer housing for rotatably driving said outer housing about the upper platform, a lower platform fixedly attached to the circumferential wall of the inner housing and outer housing; said lower platform being rotatably coupled via a ratchet mechanism to a second threaded rod comprising at least a portion of the piston shaft; said second threaded rod supported on the lower platform by a threaded nut such that rotation of said lower platform translates the piston head within the lower housing; at least one position detecting sensor disposed in the upper housing positioned proximate to either a predetermined extremity of travel of the at least one or more of the second threaded rod for detection of translation thereof, or the flap support member; and said at least one position detecting sensor operably coupled to either the piston driving means or flap driving means.

Optionally, at least one position limit detecting sensor may be disposed in the upper housing positioned proximate to the predetermined extremity of travel of the second threaded rod for detection of translation thereof; and at least one position detecting sensor is disposed in the upper housing positioned proximate to a predetermined extremity of travel of the flap support member.

At least one position limit detecting sensor may be operably coupled to at least one of the piston member driving means or the flap driving means by one or more microprocessors configured to interrupt current flow upon actuation of the sensor.

At least one position limit detecting sensor may be disposed in the upper housing is positioned proximate to the predetermined extremity of travel of the second threaded rod for detection of translation thereof and wherein the at least one position detecting sensor is actuated by detecting proximity of a protrusion on the second threaded rod.

At least one position limit detecting sensor may be disposed in the upper housing is positioned proximate to the predetermined extremity of travel of the flap support member for detection of translation thereof; and wherein said position detecting sensor is actuated by detecting proximity of a protrusion on the flap support member.

The flap driving means may be configured to extend the flaps from the space between the inner housing and outer housing in a first rotational direction and such that reversing said rotational direction of said flap driving means retracts said flaps.

The piston driving means may be configured to drive the piston to translate the piston away from the lower platform in a first rotation direction and such that reversing said rotational direction of piston driving means translates the piston toward the lower platform.

The flap support member may be translatable within the middle outer housing.

The ratchet mechanism may comprise a ratchet housing member supported on the lower platform and receiving an upper ratchet member biased into engagement with a lower ratchet member. The ratchet mechanism may include a biasing member for urging the upper ratchet member into re-engagement with the lower ratchet member.

The regolith sampling apparatus may further include at least one or more imaging acquisition devices disposed on the regolith sampling apparatus for receiving images of the operation thereof, wherein said imaging acquisition devices include one or more thermoelectric coolers coupled thereto. First and second image acquisition devices may be attached proximal to the regolith sampling apparatus; and said first image acquisition device and said second image acquisition device may be coupled via a common switch and common data cable to a common processor.

In a further aspect there a regolith sampling arm is provided. The regolith sampling arm may comprise a first regolith sampling apparatus disposed at a first end and second regolith sampling apparatus disposed at a second end.

The first regolith sampling apparatus may comprise a generally cylindrical outer housing extending about a longitudinal axis comprising upper, intermediate and lower housings; an inner housing received in said outer lower housing and comprising a circumferential wall defining a void therein, said inner housing being rotatable with the outer housing; a plurality of retractable flaps extending from between the inner housing and lower outer housing; a piston member comprising a shaft and a piston head disposed in the inner housing; a flap support member translatable along on a first threaded rod upon rotation thereof, said flap support member being coupled to said retractable flaps to urge said flaps into and out from the space between the inner housing and the lower housing upon translation of said flap support member on said threaded rod; an upper platform rotatably isolated and supported in the upper outer housing at a fixed height therein, said upper platform supporting a flap driving means rotatably coupled to the first threaded rod via a first gear train and a piston member driving means coupled to the outer housing for rotatably driving said outer housing about the upper platform, a lower platform fixedly attached to the circumferential wall of the inner housing and outer housing; said lower platform being rotatably coupled via a ratchet mechanism to a second threaded rod comprising at least a portion of the piston shaft; said second threaded rod supported on the lower platform by a threaded nut such that rotation of said lower platform translates the piston member within the middle and upper housing; at least one position detecting sensor disposed in the upper housing positioned proximate to either a predetermined extremity of travel of the at least one or more of the second threaded rod for detection of translation thereof, or the flap support member; and said at least one position detecting sensor operably coupled to either the piston driving means or flap driving means.

The second regolith sampling apparatus may comprise: a housing; a scoop member having a body for receiving regolith therein; said scoop member may be rotatable about an axle of said housing upon actuation of a scoop motor disposed in said housing; and an extendable tong configured to substantially enclose regolith received in said scoop member upon actuation of a tong motor coupled to the tong via a ratchet mechanism disposed in said housing.

The scoop member may include a plurality of serrations on the edges thereof and the extendable tong includes a plurality of teeth configured to engage with the lid of a sample container for grasping thereof.

The regolith sampling arm may further comprise one or more image acquisition devices connected via one or more thermal regulation members to one or more thermo-electric coolers.

Optionally, the regolith sampling arm may further comprise two or more image acquisition devices connected via a three state switching device with a controller.

### BRIEF DESCRIPTION OF THE DRAWINGS

In order to describe the manner in which the above-recited and other advantages and features of the disclosure can be obtained, a more particular description of the principles briefly described above will be rendered by reference to specific embodiments thereof which are illustrated in the appended drawings. Understanding that these drawings depict only exemplary embodiments of the disclosure and are not therefore to be considered to be limiting of its scope, the principles herein are described and explained with additional specificity and detail through the use of the accompanying drawings.

Preferred embodiments of the present disclosure will be explained in further detail below by way of examples and with reference to the accompanying drawings, in which:-
**FIG. 1** depicts a perspective view of an embodiment of an end effector of the present disclosure;
**FIG. 2A** depicts a partial sectional view of the first sampler depicted in **FIG. 1****.**
**FIG. 2B** depicts the views of the first motor and locations of the associated ratchets of the first sampler.
**FIG. 2C** depicts a perspective view of the shovel end member of the first sampler of **FIG. 2A** with the cover removed.
**FIG. 2D** depicts a perspective view of the shovel end member of the first sampler of **FIG. 2A****.**
**FIG. 3A** depicts a perspective view of the first sampler of the effector arm in a first mode of operation wherein the crawl is in a preparatory position before collection of samples.
**FIG. 3B** depicts a perspective view when the tong member is partially extending to enclose the sample and crawl.
**FIG. 3C** depicts a perspective view when the shovel of the first sampler of **FIG. 3A** has collected samples and has rotated partially away from the non-terrestrial surface with a sample contained therein.
**FIG. 3D** depicts a perspective view of the embodiment of **FIG. 3A** when the shovel of the first sampler with a sample ready for extension of the tong.
**FIG. 4** depicts a perspective view when the shovel of the first sampler of **FIG. 1** is in the process of sample collection of a large unitary regolith sample.
**FIG. 5A** depicts a perspective view showing a sample collected by the shovel of the first sampler in the process of depositing regolith into a container.
**FIG. 5B** depicts a perspective view of the embodiment of the first sampler of the end effector of **FIG. 1** when the tong and crawl are separated from each other in preparation for engagement with the lid of a container.
**FIG. 6A** depicts a partial sectional view of a second sampler of the end effector embodiment depicted in **FIG. 1** in an initial state.
**FIG. 6B** depicts a sectional view of the embodiment of the second sampler of **FIG. 1** when flaps are partially opened.
**FIG. 6C** depicts a sectional view of the embodiment of the second sampler of **FIG. 1** when flaps are fully opened.
**FIG. 6D** depicts a simplified sectional view of the embodiment of the flap actuating mechanism of the second sampler of **FIG. 6A****.**
**FIG. 6E** depicts a simplified sectional view of the embodiment of the piston actuating mechanism of the second sampler of **FIG. 6A****.**
**FIG. 7A** depicts a cross sectional view of the embodiment of the second sampler of the end effector depicted in **FIG. 1** when a piston is in an initial position.
**FIG. 7B** depicts a cross sectional view of the embodiment of the second sampler of the end effector depicted in **FIG. 1** when a piston is in a mid-position.
**FIG. 7C** depicts a cross sectional view of the embodiment of the second sampler of the end effector depicted in **FIG. 1** when a piston is in a fully retracted position.
**FIG. 7D** depicts a partially enlarged perspective view of the second sampler of the end effector embodiment depicted in **FIG. 1****.**
**FIG. 7E** depicts a cross sectional view of the second sampler of the end effector embodiment depicted in **FIG. 1** showing a soft stop mechanism.
**FIG. 7F** depicts a cross sectional view of the second sampler of the end effector embodiment depicted in **FIG. 1** showing a piston actuating soft stop mechanism.
**FIG. 7G** depicts a cross sectional view of the second sampler of the end effector embodiment depicted in **FIG. 1** showing a flap extension/retraction "soft stop" mechanism.
**FIG. 8A** depicts a perspective view when the second sampler of the end effector embodiment depicted in **FIG. 1** is in a sample collection mode.
**FIG. 8B** depicts a perspective view of the end effector embodiment depicted in **FIG. 1** in sample deposition mode.
**FIG. 8C** depicts a perspective view of the second sampler of the end effector embodiment depicted in **FIG. 1** engaged with the lid of a container
**FIG. 9A** depicts a perspective view of an exemplary camera of the end-effector system of **FIG. 1****.**
**FIG. 9B** depicts a perspective view of a thermal regulation member of the embodiment of the end-effector depicted in **FIG. 1****.**
**FIG. 9C** depicts a cross sectional view of the thermal regulation member of **FIG. 9B****.**
**FIG. 9D** depicts a cross sectional view showing a data transmission cable of the embodiment of the end-effector system depicted in **FIG. 1****.**

### DETAILED DESCRIPTION OF THE PREFERRED EMBODIMENTS

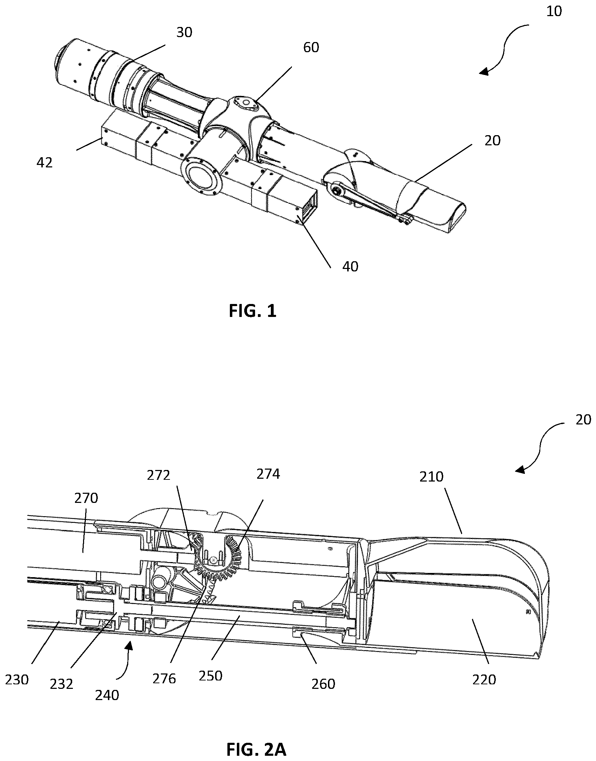

Referring to FIG. 1, there is depicted an exemplary perspective view of a robotic sampling assembly for non-terrestrial sampling according to a first embodiment of the present disclosure. As depicted, the sampling assembly **10** comprises a first end effector assembly **20** and a second end effector assembly **30.** Also visible is the outer housing **50** of first camera assembly **40** and second camera assembly **42.** These camera assemblies are located on either side of the central axle **60** to which it would be appreciated an elongate arm may be attached (not shown). Rotation of the first end effector assembly **20** and second end effector assembly **30** about the central axle can be monitored by the attached camera assembly **40** and **42.**

Depending on the type of regolith sample which is to be collected, the sample may be collected using the first end effector assembly **20** or the second end effector assembly **30.** It would be appreciated that for loose regularly sized small rocks/coarse-grained soil, the first end effector assembly **20** is particularly useful as this type of material is easily collected by the mechanism depicted. Conversely for collecting regolith of fine-grained soil which is sticky, with semi-rigid dense and high strength, the second end effector assembly **30** would be particularly useful, principally operating using a rotational coring type approach.

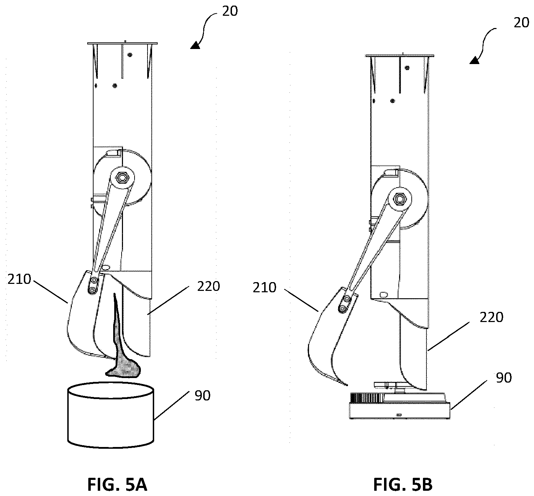

Now referring to FIGS. 2A-2D, the first end effector sampler **20** is discussed. As depicted, the first end sampler **20** comprises a scoop **210** and a tong **220** for collecting regolith sample. The scoop **210** is able to rotate around scoop axle **214** via elongate member **212** for scooping up regolith samples from the ground. The tong **220** is extendable from a retracted position wherein the tong **220** is received within a housing of the first end sampler **20** to an extended position in which the tong **220** fully extends over and encloses the cavity of the scoop **210.** When covering the scoop the regolith sample is trapped inside the first end effector **20** and may be conveyed by the main arm (not shown) to a sample container **90** (e.g. depicted in FIG. 5A). In addition the tong **220** may engage with the lip of the lid of the sample container **90** as depicted in FIG. 5B, to extract the sealed container from the sampling apparatus.

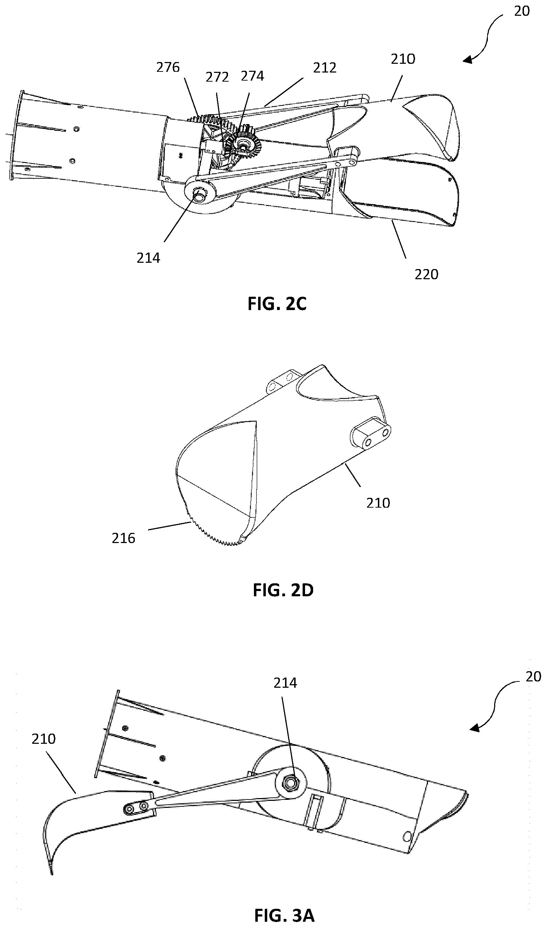

As would be appreciated by persons skilled in the art, an arrangement of multiple ratchet members such as that depicted in FIG. 2D produces a range of vibrational movements, the amplitude of which is determined by the relative sizing and number of steps of adjacent ratchet members. Once the adjacent ratchets are engaged with each other, rotation of the motor drives the lead screw nut (and hence translates the tong) with no vibration.

The first end effector sample **20** can provide the tong with three working modes, i.e., an extending mode, a rough vibration mode, a gentle vibration mode.

Referring particularly to FIG. 2A, to extend the tong **220**, a first motor **230** drives a pinon gear **232** coupled via a lead screw ratchet set **240** to drive a lead screw **250.**

When the tong is able to extend (without any rock jammed between the tong and the scoop), rotation of the lead screw **250** drives lead nut **260** to translate along the lead screw **250.**

Lateral movement of the lead screw nut **260** along the lead screw **250** urges the attached tong **220** in a direction of away from the first motor **230** to extend and cover the cavity of the scoop **210.** (Accordingly, it would be appreciated that reversing the direction of rotation of the lead screw relative to the lead screw nut in turn retracts the tong **220** into the housing to uncover the cavity of the scoop **210**).

The stopper ratchet **240c** is fixed at a predetermined lateral position in the end effector sample **20** and rotates together with the lead screw **250.** The intermediate ratchet **240b** and the stopper ratchet **204c** are engaged with each other and do not move apart from each other under normal operation.

The tong **220** may become stuck, for example by being urged against regolith (e.g. a rock) projecting out of the scoop during as it is extending over the scoop. As there is no other means to clear the rock from the tong (e.g. no other person or other mechanism will be nearby) it is critical to provide a mechanism to produce a "juddering" mechanism which vibrationally shakes the tong **220** to clear any blocking regolith.

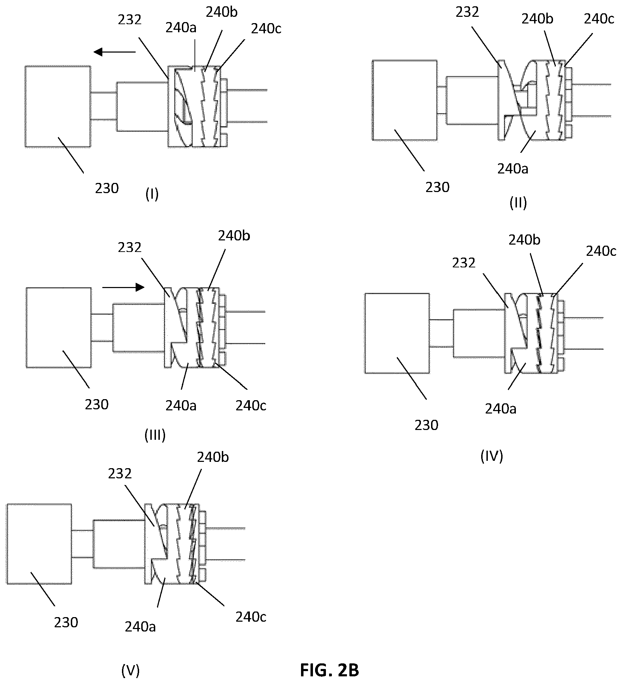

These vibrations of the tong **220** are provided by coupling the first motor **230** via a pinion gear **232** to a ratchet set **240** (including a lead screw ratchet **240a**, an intermediate ratchet **240b** and a stopper ratchet **240c**) as shown in detail in FIG. 2B.

A "big jolt" would only be triggered when the tong **220** and connected lead screw ratchet **240a** is forced to stop due to getting stuck while the motor **230** keeps rotating in normal direction the pinion gear **232.**

When the extension of the tong is blocked by a regolith, the force applied on the tong **220** exceeds a predetermined threshold value. Detection of this value causes the motor to reverse direction, pulling the pinion gear **232** away from the lead screw ratchet **240a** as depicted in (I) and (II) of FIG. 2B.

When the first motor **230** drives the pinion gear **232** to rotate in normal direction, the face of the pinion gear **232** which includes a large step and an inclined surface is urged into engagement with a corresponding surface on the lead screw ratchet **240a** under biasing forces provided by a resiliently deformable member (e.g. spring). This resiliently deformable member (not shown) may be located within the housing of the pinion gear **232** so as to urge the ratchet set **240** together, as depicted in (III) of FIG. 2B.

In the gentle vibration mode, the intermediate ratchet **240b** is locked by the stopper ratchet **240c** and therefore is not able to rotate with lead screw ratchet **240a.** The lead screw ratchet **240a** and the intermediate ratchet **240b** are able to disengage from each other while the lead screw ratchet **240a** and the pinion **232** are engaged as depicted in (III) of FIG. 2B.

Similarity, via the spring located within the housing of the pinion **232**, the rotation of the pinion **232** (and the lead screw ratchet **240a**) can urge the surface of lead screw ratchet **240a** to jump into a corresponding shoulder formed as shown in the mating surface of the intermediate ratchet **240b**, which provides a gentler vibration of the lead screw, as depicted in (IV) of FIG. 2B.

It depicts the motor **230** rotates in the reverse direction and drives the pinion gear **232** to couple with lead screw ratchet **240a** which in turn couples with the adjacent intermediate ratchet **240b** so that all the three components (**232**, **240a** and **240b**) rotates together in the reverse direction to retract the tong **220** and gentle vibration results due to the stepping with the stopper ratchet **240c.**

Now referring FIGS. 2A and 2C, the rotational movement of the scoop **210** about the scoop axle **214** is implemented via a second motor **270.** As shown the second motor **270** drives a pinion gear **272** which in turn rotates a bevelled intermediate gear **274.** Rotation of the bevelled intermediate gear drives the swivel gear **276** to which the scoop arm **212** is attached. The scoop arm **212** and attached scoop **210** can then be rotated away from and towards the tong **220** as depicted in FIG. 2C. The same movement of the scoop **210** towards/away from the tong **220** can be used for the sample deposition as depicted FIG. 5A into sample container **90**, or engagement with the lid of the sample container **90** depicted in FIG. 5B. As depicted in FIG. 2D, the scoop **210** can be configured with a toothed edge **216** for facilitating collection of regolith.

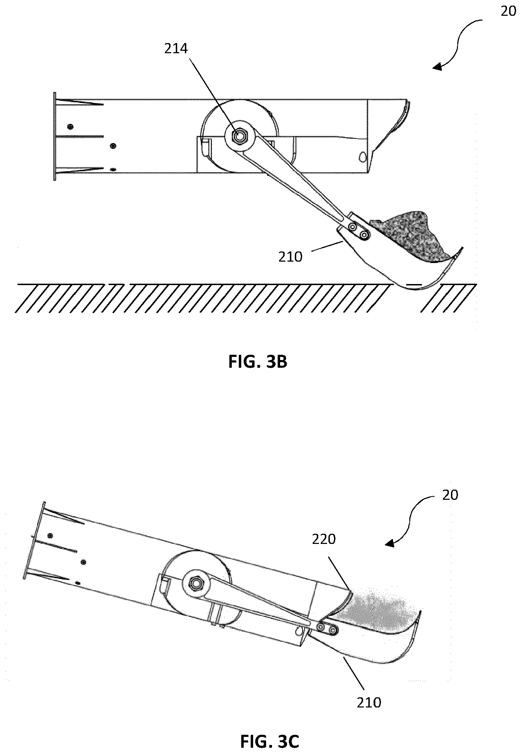

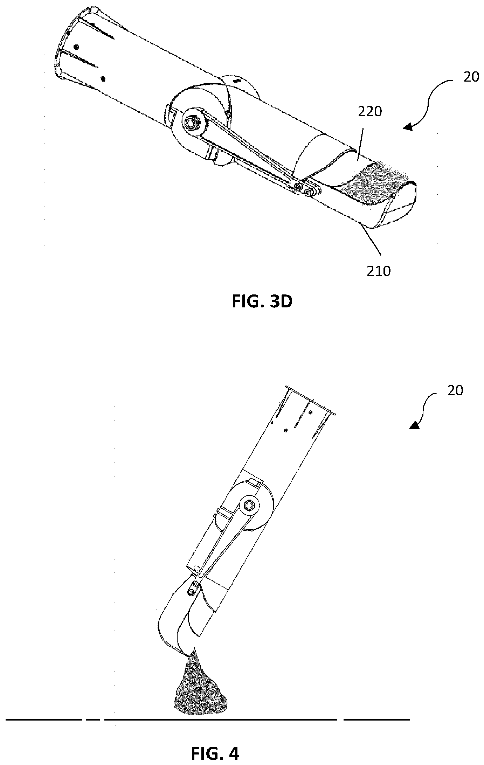

FIGS. 3A and 3B show various positions of the scoop **210** relative to the end effector **20** for collection of regolith. FIGS. 3C and 3D show exemplary positions of the tong **220** extending over collected sample to partially enclose this sample as described above.

FIG. 4 depicts an alternate mode of operation in which the end effector **20** is inclined relative to the surface for capturing the scoop **210** a portion of projecting regolith. This sampling operations proceeds via the coordinated action of the scoop **210** and tong **220** of the first end effector **20** as described above, using a combination of rotation of the scoop **210** and extension of the tong **220.**

FIG. 5A depicts the scoop **210** rotating away from the tong **220** of the first end effector **20** to deposit the regolith sample in the container **90** in accordance with the operational principles described above.

FIG. 5B depicts cooperation between the scoop **210** and tong **220** of the first end effector **20** to locate on the lip of the lid of a sealed sample container **90** for removal from the sampling assembly and transfer to the launch vehicle (not shown) for return to Earth.

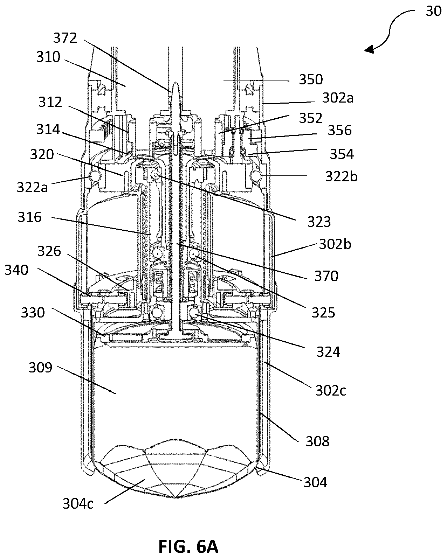

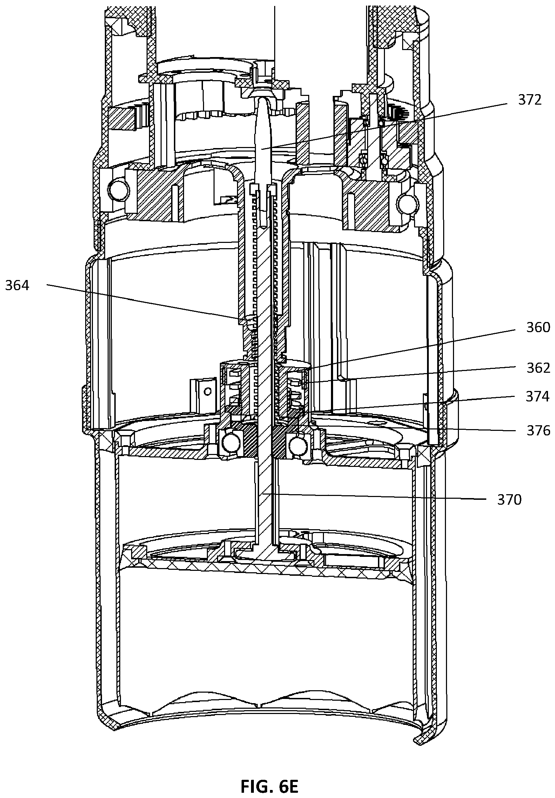

Referring to FIGS. 6A-6D, there is depicted an exemplary cross-sectional view of the second end effector assembly **30** according to an embodiment of the present disclosure, in relation to which the mechanisms for extension and retraction of flaps **304** and for movement of a piston member are described.

Multiple flaps **304a**, **304b**, **304c**, **304d**, **304e**, **304f**, **304g**, **304h** (collectively as **304**, a selection only being depicted) extend from between the lower portion of the outer housing **302c** as shown In FIG. 6A. These flaps are retained between the lower portion of the outer housing **302c** and an inner chamber housing **308.** The inner chamber housing contains a void **309** in which the regolith sample is received. The piston member includes a piston lead screw **370** (comprising or engaged with at least a portion of a piston shaft) and a piston head 330 located at the end of the piston shaft.

There are two main operational aspects of the second end effector assembly **30** depicted in FIGS. 6A-6D; first the extending and retracting flaps **304a-h** for collecting the regolith sample and second the operation of the piston head **330** to expel the regolith sample from the void **309** for deposition of a regolith sample into a sampling container (this sampling container is then returned back to earth in the launch vehicle for analysis).

In general, as will be described further herein, the second end effector assembly **30** comprises an outer housing **302** made up of upper, middle and lower portions **302a**, **302b**, **302c**.

The second end effector assembly **30** comprises a flap actuating mechanism **300a** and a piston actuating mechanism **300b.** The fixed upper platform **320** is rotatable around a fixed height of the upper housing **302a**; an upper platform **320** attaches the second end effector sampler **30** to a robot arm (not shown). Outer ball bearings **322** (respectively referred to as **322a** and **322b**) are for isolating the relative rotation between the outer housing **302** and the upper platform **320.** Inner upper ball bearings **323** and inner intermediate ball bearings **325** are used to isolate the rotation of the upper platform **320** from the rotation of a first lead screw **316** of the flap actuating mechanism **300a.**

Referring to FIGS. 6A-6D a third motor **310** is rotatably coupled with a pinion gear **312.** The teeth of the pinion gear **312** mesh with upper teeth of intermediate gear **314.** Hence, rotation of the third motor **310** in either clockwise or anticlockwise directions rotates the intermediate gear **314** and in turn rotates the toothed portion of the flap lead screw **316** which is disposed external of the movable piston member. The flap lead screw **316** extends down from the fixed upper platform support **320** into the rotatable middle portion **302b** of the outer housing.

The flap lead nut **326** is movably driven by the flap lead screw **316** to rotate about the lead screw **316**; thereby moving up and down within the middle portion **302b** of the outer housing **302.**

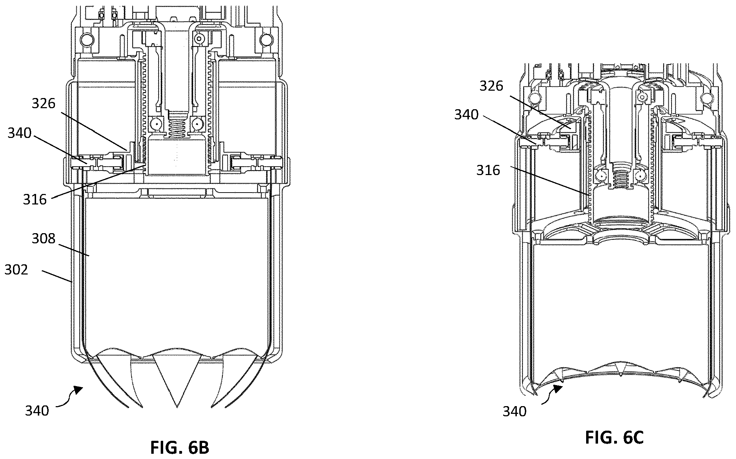

As the flap lead screw is attached to the flap holder **340**, the position of the flap holder relative to the middle portion of the outer housing also changes. Specifically, rotation of the lead screw in turn drives the lead nut which in turn translates the flap holder **340** upwardly from the position depicted in FIG. 6A through the position depicted in FIG. 6B to the final position depicted in FIG. 6C.

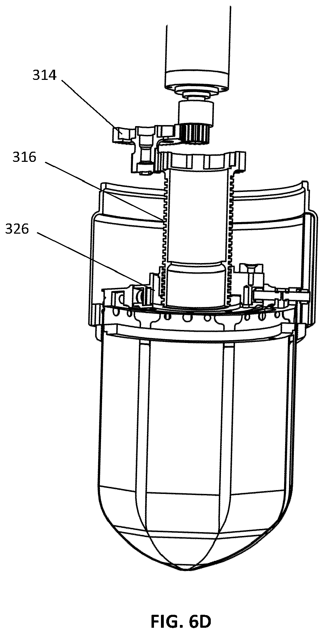

As the flaps **304a-h** are attached with the flap holder **340**, the translation of the flap holder upwards as depicted in FIGS. 6A to 6D also moves the flaps **304a-h** upwards; retracting the flaps into recesses (not shown) between the inner housing **308** and the outer housing **302**, especially **302c.** FIG. 6D depicts a simplified sectional view of the embodiment of the second sampler of FIG. 6D without the outside housing **302c** and the piston actuating mechanism for movement of the piston head **330** via translation of the attached piston shaft **370.**

As would be appreciated by persons skilled in the art, reversing the direction of rotation the third motor **310** in turn reverses the direction of rotation of the lead screw **316** and hence reverses the direction of travel of the flap lead nut **326** inside the outer housing **302.** In this way, the extension and retraction of the flaps during the sampling operation of the second end effector assembly **30** is achieved.

When the second end effector sampler **30** is actuated by the third motor **310** (flap extension/retraction) or the fourth motor **350** (piston moving upwardly and downwardly as will be discussed below), the upper platform **320** does not change in the relative position in the outer housing **302.**

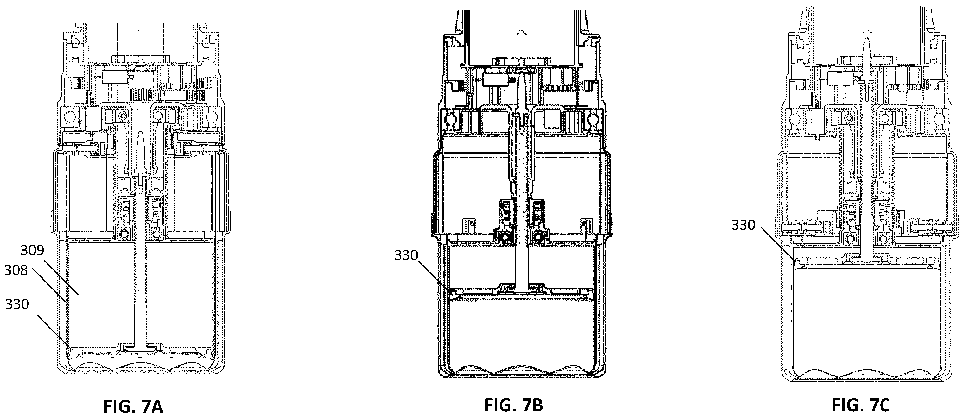

Referring now to FIGS. 7A to 7C, during the sample deposition process, regolith sample which is contained in the void **309** of the inner chamber housing **308** is urged out of the void, into a sample container (not shown).

At launch, for maximum stability the piston head **330** is in a fully down position, as depicted in FIG. 7A. After the flaps are in a fully open positon as described with reference to FIGS. 6A-6D, the piston head **330** moves through the intermediate position depicted in FIG. 7B into a fully up position as depicted in FIG. 7C. Once sampling has occurred, to expel the regolith contained in the void **309**, the piston head **330** needs to move back to the fully down position depicted in FIG. 7A through the intermediate position of FIG. 7B.

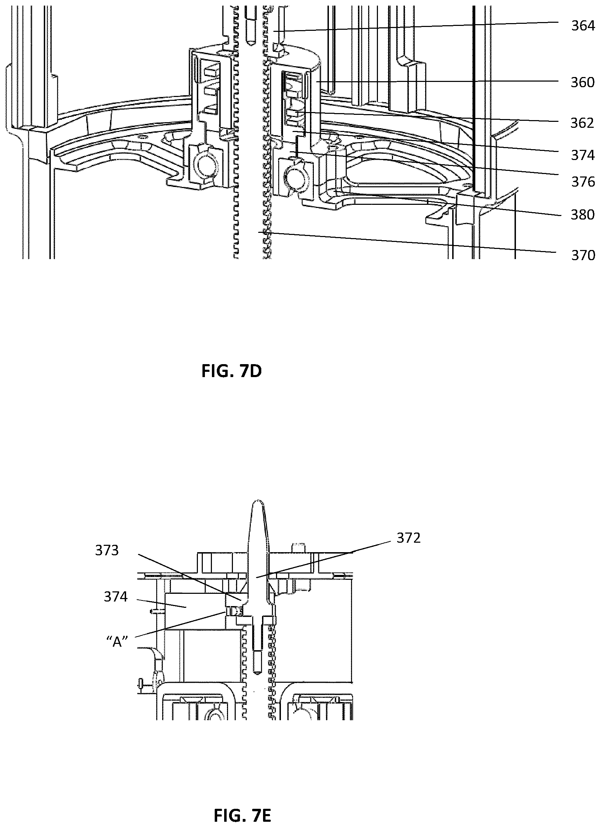

In order to achieve reliable upward and downward movement of the piston head as described, rotational motion of fourth motor **350** is converted to translation of the piston head in the housing **302c.** Referring to FIG. 6A and FIG. 7D, translation of the piston head **330** is driven by the fourth motor **350** via rotation of the second pinion gear **352**, which in turn rotates the second intermediate gear **354.**

Rotation of the second intermediate gear **354** rotates the outer ring gear **356** which is engaged with the outer housing **302** which in turn rotates relative to the motor via the outer ball bearings **322**.

In detail, rotation of the fourth motor **350** is coupled via a second pinion gear **352** to drive rotation of the lower teeth of second intermediate gear **354** to bear against the outer ring gear **356** which is affixed to the upper portion of the outer housing **302.**

In this way, the external housing **302** is driven to rotate relative to the upper platform assembly **320.** The outer ball bearings **322a**, **322b** isolate the rotation of the outer housing **302** from the upper platform **320.** The inner housing **308** has a platform **380** which is attached to the outer housing **302.** The rotation of the outer housing **302** drives the inner housing **308** to rotate.

The upper ratchet housing **360** fixed on the platform **380.** The ratchet housing **360** contains an upper ratchet **374** which has a protrusion receivable in a corresponding recess on inner side wall of the housing **360.** The upper ratchet **374** is thereby coupled with a lower ratchet **376.**

The rotation from the outer housing **302** and inner housing **308** is transmitted to the upper ratchet housing **360** (which is in turn connected with the upper ratchet **374**) which is capable of coupling with the lower ratchet **376.** Thus the rotation of the outer housing results in translation of the lead screw **370** (piston shaft) which is connected to the piston head inside the housing.

Inner lower ball bearings **324** are retained between the ratchet housing **360** and the platform **380** and isolate the lower ratchet **376** from the rotation of the platform **380.**

The lower ratchet **376** has a protrusion which is receivable in a corresponding recess in the upper ratchet **374.** The upper ratchet **374** and lower ratchet **376** enable relative rotation and bouncing between the two parts while allowing transmission of torque to drive the lead screw. This torque drives rotation of the piston lead screw **370** and converts to translation via second lead nut **364**, retained on the upper platform **320.** It would be appreciated that the relative position of the piston lead nut **364** relative to the intermediate outer housing **302b** does not change in height; although it rotates.

As the regolith may adhere to the surfaces of the moving parts (such as piston head **330**, inner chamber housing **308**, and platform **380**), the regolith adhering to the parts needs to be removed during the sample collection to provide smooth operation.

Jamming of the sampling apparatus on a regolith sample in the middle of the sampling process must be avoided as it could potentially jeopardise the success of the sampling mission as there is no possibility of human intervention. As a result, the sampling mission cannot be completed in the event of jamming.

To prevent jamming due to regolith, a shaking operation is provided and starts by the rotation of the ratchet housing **360** which transmits torque to rotate the upper ratchet **374** while the lower ratchet **376** gets stuck. A spring **362**, is provided in the ratchet housing **360**, which preloads the complementary inclined surfaces of the upper ratchet **374** against the lower ratchet **376** into engagement with each other. In normal operation, the rotation of the upper ratchet and the lower ratchet provide urge the spring **362** into compression. In order to remove the adhered regolith, the spring **362** urges the upper ratchet and the lower ratchet to jump downwardly so as to bump off the regolith adhered to the parts.

Rotation of the piston lead screw **370** in the piston lead nut **364** results in translation of the piston lead screw **370** (shaft) axially; which moves the attached piston head **330** axially within a portion of the inner housing **308**, which is disposed in the lower portion of the outer housing **302.**

A tapered trigger member **372** is attached to the top end of the second lead screw **370** and moves with the translating of piston lead screw **370.**

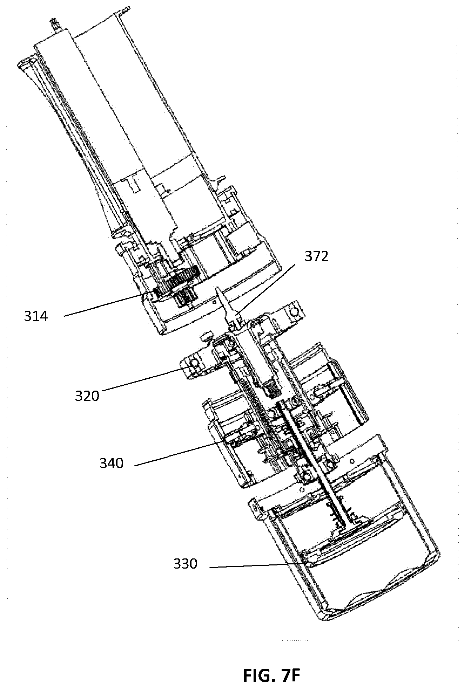

As shown in FIG. 7E and FIG. 7F, when the piston lead screw **370** and the piston head **330** move upwards proximal to a limit position, a protruding shoulder **373** of the tapered trigger member **372** is brought near to a triggering position marked "A". Once the shoulder **373** reaches the triggering position, the shoulder **373** of the tapered trigger member **372** actuates a limit switch **374** which is configured to break the circuit driving the fourth motor **350.** Actuation of the limit switch in this way ensure the extremity of movement of the piston lead screw **370** relative to the upper platform **320** can be reliably and quickly detected.

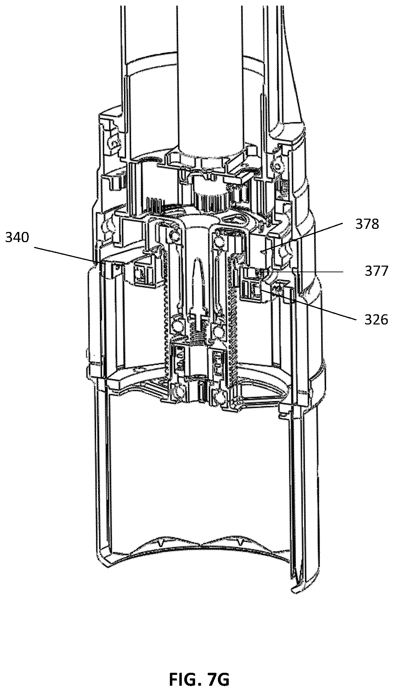

It would be appreciated that a similar limiting configuration of flap limit switch **378** (as shown in FIG. 7G) can be used for limiting the position of the flap lead nut **326** (as well as the attached flap holder **340**) within the intermediate outer housing **302b.** When the flap lead nut **326** and the flap holder **340** approach the limit position by rotating on the flap lead screw **316**, the flap holder **340** approaches an actuator **377** of the flap limit switch **378.**

Once the holder **40** reaches the limit position, the actuator **377** operates the flap limit switch **378** (either mechanically, optically, magnetically, or similar as would be appreciated by persons skilled in the art). This switch may in turn be directly coupled (or indirectly coupled via a signal to a processor) to break the power circuit providing power to the third motor **310**; thereby stopping its operation.

It would be appreciated that even if the control centre on earth is in communication with the remote lander but, due to the time delay in signals travelling between the mission control and the remotely located lander, it is not possible to observe and/or control the operation of the regolith sampling device in a timely manner during its operations. Therefore it is critical that the operation of flap extension and piston extension mechanism are controlled independently within predetermined limits using the immediate information received from the position detecting/monitoring sensors.

The operation of the position limit detecting sensors thus operate to stop the travel of the flap lead nut **326** on the flap lead screw **316**; and hence prevent any further retraction of the flaps/flap member.

As compared to physical mechanisms which may be used to limit the travel of the second lead screw **370** (the piston actuating mechanism) relative to the upper platform **320** or the flap lead nut **326** on the flap lead screw **316**; the respective sensors act as "soft" position limits. This arrangement provides buffer time and extra distance so that before arriving at any physical limit positions (e.g. traveling beyond the threads of the respective lead screw), the piston actuating mechanism and the flap actuating mechanism cease operation safely after power supply to the respective motor is cut-off. This cessation in operation is performed relatively smoothly, without abrupt impact that would damage the delicate part(s), as would be the result of a mechanical stop.

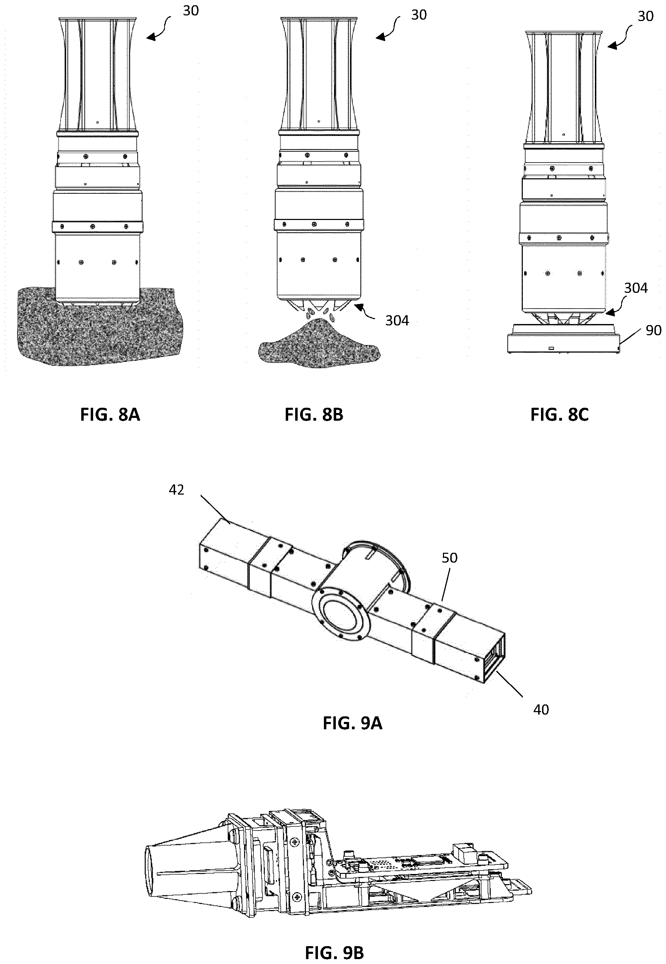

FIGS. 8A to 8C depicts various operational functions which may be performed by the second end effector sampler **30.**

Specifically, as depicted in FIG. 8A the flaps **304** are slightly protruding (although may also be fully extended) to assist with collection of sample regolith together with the rotation of flap holder **340** in the lower portion of the outer housing **302c.**

As depicted in FIG. 8B, once a suitable depth has been reached, the flaps may be retracted as shown and described above to close off the void **309**; and as shown the second end effector sampler **30** may be withdrawn from the sampling region by movement an arm (not shown) to which said end effector sampler **30** is attached.

As depicted in FIG. 8C, once the regolith sample has been deposited in the sampling container **90**, and the lid **91** secured; the filled sampling container needs to be extracted from the landing craft and inserted into the space craft which will return the sample to Earth. This operation may be accomplished by engagement of the flaps **304** with a locating rim of the lid **91** as depicted.

Now referring to FIG. 9A there is depicted a perspective view of an exemplary pair of cameras **40**, **42** which are disposed in a housing **50** and on either side of central axle **60** as shown.

Referring to FIG. 9B the housing **50** has been removed. An exemplary camera image acquisition device can be CCDs, CMOS, and thermal regulation members.

Advantageously, more than one thermoelectric coolers (TECs) can be used to utilise the Peltier effect which draws electric current and actively pump heat from a first surface (cold surface **394a**, **395a**) to second surface (hot surfaces **394b**, **395b**). As a result, the temperature at the cold surface are lower than that of the hot surfaces.

The cold surfaces **394a**, **395a** of the TECs are attached to the contact surface with the heat generating components (heat sources), such as CMOS image sensors 392 and FPGAs **396**.

The corresponding hot surfaces **394b**, **395b** are attached to the contact surface of the thermal regulation members (heat sinks).

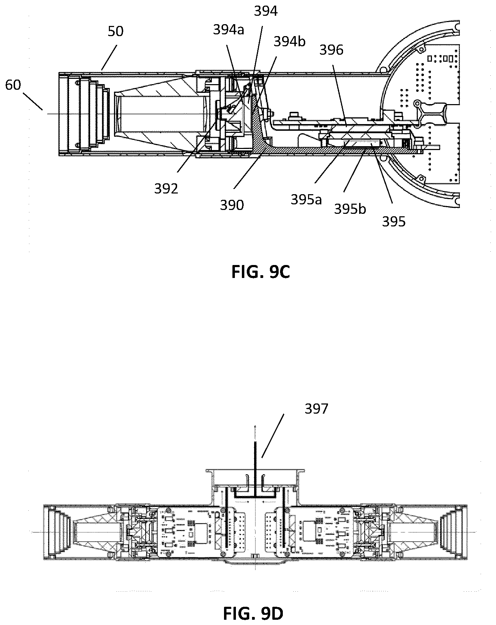

As is known in the art, a TEC has only two wires (i.e. 1 red and 1 black) to draw current from an electrical power supply to create a temperature difference between the hot side and cold side. The hot and cold sides are marked in FIG. 9C.The TEC has many advantages for temperature regulation, providing a high accuracy and stability, fast response time, a wide set-point temperature range and at the same time provide a simple structural design without moving parts, small and lightweight, reliable, noise-free and environmentally friendly.

The thermal regulation member inside the camera is made up of lightweight but high stiffness and high thermal conductivity material to protect the fragile optics and electronics components while transfer the heat efficiently and effectively to the outer casing.

Accordingly, a heat transfer path is provided to transfer heat generated from the heat sources via the TECs to the connected camera casing and then further transferred to the camera outer surface where it is released.

As shown in FIG. 9D, two sets of data transmission cables **397** of the two cameras are connected to an electronic tri-state switching device as a switch which enables automatic camera selection and a common communication bus for data communication in both directions. Since the device only needs one set of cables connected to the controller side, the total number of cables are significantly reduced and are able to pass through the very limited narrow internal routing passage in the robot arm which is provided, thereby minimising weight and size required.

The disclosure provides a more reliable regolith collecting system used remotely away from potential intervention in the case of malfunction (e.g. caused by regolith jamming in the various mechanisms or simply starting/stopping of various stages in the operation).

Advantageously, the sensor based limit detection described herein enables smooth deceleration of moving components such as the flaps and piston mechanism. This avoids physical damage resulting from abrupt contact which could be caused if mechanical constraints were used to restrain movement beyond extremities of the desired range.

It would be appreciated that if a mechanical stop were utilised, the motor may continue to provide power for some time after the extremities of travel have been exceeded. Mechanical stop arrangements therefore pose a significantly higher risk of jamming or mechanical failure, which cannot be rectified easily due to the remoteness of operation. Therefore such mechanical arrangements increase the risk that the regolith retrieval operation may fail.

Accordingly, the "soft stop" configuration of an aspect of the present disclosure provides a more reliable system which significantly reduces the potential risks of jamming or malfunction as would be the case if alternative mechanical position limiting arrangements were utilised.

In addition, the TEC described herein provides a high accuracy and stability, fast response time, a wide set-point temperature range and simple structural design without moving parts, and are small and lightweight, reliable, noise-free and environmentally friendly.

The two sets of data transmission cables of the two cameras provide a high speed transmitting of image data. Accordingly, the above advantages individually and in combination contribute to a more reliable regolith sampling system without adding significant additional weight or volume.

The above embodiments are described by way of example only. Many variations are possible without departing from the scope of the disclosure as defined in the appended claims.

## Claims

1. A regolith sampling apparatus, comprising:
a cylindrical outer housing (302) extending about a longitudinal axis comprising upper (302a), intermediate (302b) and lower housings (302c);
an inner housing (308) received in said outer lower housing and comprising a circumferential wall defining a void (309) therein, said inner housing being rotatable with the outer housing;
a plurality of retractable flaps (304) extending from between the inner housing (308) and lower outer housing (302c);
a piston member comprising a piston shaft (370) and a piston head (330) disposed in the inner housing;
a flap support member (340) translatable along on a first threaded rod (316) upon rotation thereof, said flap support member (340) being coupled to said retractable flaps (304) to urge said flaps into and out from the space between the inner housing (308) and the lower housing (302c) upon translation of said flap support member (340) on said threaded rod;
an upper platform (320) rotatably isolated and supported in the upper outer housing (302a) at a fixed height therein, said upper platform supporting a flap driving means (310) rotatably coupled to the first threaded rod via a first gear train (312) and a piston member driving means (350) coupled to the outer housing for rotatably driving said outer housing (302) about the upper platform (320),
a lower platform (380) fixedly attached to the circumferential wall of the inner housing and outer housing; said lower platform (380) being rotatably coupled via a ratchet mechanism (360) to a second threaded rod comprising at least a portion of the piston shaft (370); said second threaded rod supported on the lower platform (380) by a threaded nut such that rotation of said lower platform translates the piston head (330) within the lower housing;
at least one position limit detecting sensor (378) disposed in the upper housing (302a) positioned proximate to either a predetermined extremity of travel of the at least one or more of the second threaded rod for detection of translation thereof, or the flap support member (340); and
said at least one position detecting sensor (378) operably coupled to either the piston member driving means (350) or flap driving means (310).

2. The regolith sampling apparatus of claim 1, wherein at least one position limit detecting sensor (378) is disposed in the upper housing (302a) positioned proximate to the predetermined extremity of travel of the second threaded rod for detection of translation thereof; and at least one position detecting sensor (378) is disposed in the upper housing positioned proximate to a predetermined extremity of travel of the flap support member.

3. The regolith sampling apparatus of claim 1, wherein at least one position limit detecting sensor (378) is operably coupled to at least one of the piston member driving means (350) or the flap driving means (310) by one or more microprocessors configured to interrupt current flow upon actuation of the sensor (378).

4. The regolith sampling apparatus of claim 1, wherein at least one position limit detecting sensor (378) disposed in the upper housing (302a) is positioned proximate to the predetermined extremity of travel of the second threaded rod for detection of translation thereof and wherein the at least one position detecting sensor is actuated by detecting proximity of a protrusion (372) on the second threaded rod.

5. The regolith sampling apparatus of claim 1, wherein the at least one position limit detecting sensor (378) disposed in the upper housing (302a) is positioned proximate to the predetermined extremity of travel of the flap support member (340) for detection of translation thereof; and wherein said position detecting sensor is actuated by detecting proximity of a protrusion on the flap support member.

6. The regolith sampling apparatus of claim 1, wherein if said flap driving means (310) is configured to extend the flaps (304) from the space between the inner housing and outer housing in a first rotational direction and reversing said rotational direction of said flap driving means retracts said flaps.

7. The regolith sampling apparatus of claim 1, wherein if said piston driving means (350) is configured to drive the piston shaft (370) to translate the piston head (330) away from the lower platform in a first rotation direction and reversing said rotational direction of piston driving means translates the piston head (330) toward the lower platform.

8. The regolith sampling apparatus of claim 1, wherein the flap support member (340) is translatable within the middle outer housing 302(b).

9. The regolith sampling apparatus of claim 1, wherein the ratchet mechanism (360) comprises a ratchet housing member supported on the lower platform and receiving an upper ratchet member (374) biased into engagement with a lower ratchet member (376), the ratchet mechanism preferably includes a biasing member for urging the upper ratchet member (374) into re-engagement with the lower ratchet member (376).

10. The regolith sampling apparatus of claim 1, further including at least one or more imaging acquisition devices (40, 42) disposed on the regolith sampling apparatus for receiving images of the operation thereof, wherein said imaging acquisition devices include one or more thermoelectric coolers (394a, 394b, 395a, 395b) coupled thereto.

11. The regolith sampling apparatus of claim 10, wherein a first and second image acquisition devices (40, 42) are attached proximal to the regolith sampling apparatus; and wherein said first image acquisition device (40) and said second image acquisition device (42) are coupled via a common switch and common data cable to a common processor.

12. A regolith sampling arm comprising a first regolith sampling apparatus (30) according to any of claims 1-11 disposed at a first end and second regolith sampling apparatus (20) disposed at a second end;
said second regolith sampling apparatus (20) comprising:
a housing;
a scoop member (210) having a body for receiving regolith therein; said scoop member being rotatable about an axle of said housing upon actuation of a scoop motor disposed in said housing; and
an extendable tong (220) configured to substantially enclose regolith received in said scoop member upon actuation of a tong motor (230) coupled to the tong via a ratchet mechanism disposed in said housing.

13. The regolith sampling arm of claim 12, wherein at least one of the scoop member (210) or the extendable tong (220) includes a plurality of serrations on the edges thereof.

14. The regolith sampling arm of claim 12 further comprising one or more image acquisition devices (40, 42) connected via one or more thermal regulation members (394a, 394b, 395a, 395b) to one or more thermo-electric coolers.

15. The regolith sampling arm of claim 12 further comprising two or more image acquisition (40, 42) devices connected via a three state switching device with a controller.

## Patentansprüche

1. Vorrichtung zum Beproben von Regolith, Folgendes umfassend:
ein zylinderförmiges äußeres Gehäuse (302), das sich um eine Längsachse erstreckt und ein oberes (302a), ein mittleres (302b) und ein unteres Gehäuse (302c) umfasst,
ein inneres Gehäuse (308), das in dem äußeren unteren Gehäuse aufgenommen ist und eine Umfangswand umfasst, die in sich einen Hohlraum (309) definiert, wobei das innere Gehäuse mit dem äußeren Gehäuse drehbar ist,
mehrere einziehbare Klappen (304), die sich von zwischen dem inneren Gehäuse (308) und dem unteren äußeren Gehäuse (302a) erstrecken,
ein Kolbenelement, das einen Kolbenschaft (370) und einen Kolbenkopf (330) umfasst und in dem inneren Gehäuse angeordnet ist,
ein Klappenträgerelement (340), das entlang einer ersten Gewindestange (316) auf eine Drehung derselben hin verlagerbar ist, wobei das Klappenträgerelement (340) mit den einziehbaren Klappen (304) gekoppelt ist, um auf das Verlagern des Klappenträgerelements (340) an der Gewindestange hin die Klappen in den Raum und aus dem Raum zwischen dem inneren Gehäuse (308) und dem unteren Gehäuse (302c) zu treiben,
eine obere Plattform (320), die drehbar isoliert ist und in dem oberen äußeren Gehäuse (302a) auf einer festen Höhe darin getragen wird, wobei die obere Plattform ein Klappenantriebsmittel (310) trägt, das über ein erstes Zahnradgetriebe (312) drehbar mit der ersten Gewindestange gekoppelt ist, und ein Kolbenelement-Antriebsmittel (350), das mit dem äußeren Gehäuse gekoppelt ist, um das äußere Gehäuse (302) drehend um die obere Plattform (320) anzutreiben,
eine untere Plattform (380), die fest an der Umfangswand des inneren Gehäuses und des äußeren Gehäuses angebracht ist, wobei die untere Plattform (380) über einen Schaltradmechanismus (360) drehbar mit einer zweiten Gewindestange gekoppelt ist, die mindestens einen Abschnitt des Kolbenschafts (370) umfasst, wobei die zweite Gewindestange durch eine Gewindemutter derart auf der unteren Plattform (380) getragen wird, dass eine Drehung der unteren Plattform den Kolbenkopf (330) in dem unteren Gehäuse verlagert,
mindestens einen Sensor (378) zur Positionsbegrenzungserkennung, der in dem oberen Gehäuse (302a) angeordnet ist und nahe entweder einem festgelegten Endpunkt der Bewegung von mindestens einem oder mehreren von der zweiten Gewindestange zum Erkennen von deren Verlagerung oder dem Klappenträgerelement (340) positioniert ist, und
wobei der mindestens eine Sensor (378) zur Positionserkennung funktionsfähig mit entweder dem Kolbenelement-Antriebsmittel (350) oder dem Klappenantriebsmittel (310) gekoppelt ist.

2. Vorrichtung zum Beproben von Regolith nach Anspruch 1, wobei mindestens ein Sensor (378) zur Positionsbegrenzungserkennung in dem oberen Gehäuse (302a) angeordnet und nahe dem festgelegten Endpunkt der Bewegung der zweiten Gewindestange zur Erkennung von deren Verlagerung positioniert ist und mindestens ein Sensor (378) zur Positionserkennung in dem oberen Gehäuse angeordnet und nahe einem festgelegten Endpunkt der Bewegung des Klappenträgerelements positioniert ist.

3. Vorrichtung zum Beproben von Regolith nach Anspruch 1, wobei mindestens ein Sensor (378) zur Positionsbegrenzungserkennung durch einen oder mehrere Mikroprozessoren, die dafür konfiguriert sind, auf eine Betätigung des Sensors (378) hin den Stromfluss zu unterbrechen, funktionsfähig mit mindestens einem von dem Kolbenelement-Antriebsmittel (350) oder dem Klappenantriebsmittel (310) gekoppelt ist.

4. Vorrichtung zum Beproben von Regolith nach Anspruch 1, wobei mindestens ein Sensor (378) zur Positionsbegrenzungserkennung, der in dem oberen Gehäuse (302a) angeordnet ist, nahe dem festgelegten Endpunkt der Bewegung der zweiten Gewindestange zur Erkennung von deren Verlagerung positioniert ist und wobei der mindestens eine Sensor zur Positionserkennung durch Erkennen der Nähe eines Vorsprungs (372) an der zweiten Gewindestange betätigt wird.

5. Vorrichtung zum Beproben von Regolith nach Anspruch 1, wobei der mindestens eine Sensor (378) zur Positionsbegrenzungserkennung, der in dem oberen Gehäuse (302a) angeordnet ist, nahe dem festgelegten Endpunkt der Bewegung des Klappenträgerelements (340) zur Erkennung von dessen Verlagerung positioniert ist und wobei der Sensor zur Positionserkennung durch Erkennen der Nähe eines Vorsprungs des Klappenträgerelements betätigt wird.

6. Vorrichtung zum Beproben von Regolith nach Anspruch 1, wobei, wenn das Klappenantriebsmittel (310) dafür konfiguriert ist, die Klappen (304) aus dem Raum zwischen dem inneren Gehäuse und dem äußeren Gehäuse in einer ersten Drehrichtung auszufahren, und das Umkehren der Drehrichtung des Klappenantriebsmittels die Klappen einzieht.

7. Vorrichtung zum Beproben von Regolith nach Anspruch 1, wobei, wenn das Kolbenantriebsmittel (350) dafür konfiguriert ist, den Kolbenschaft (370) anzutreiben, den Kolbenkopf (330) weg von der unteren Plattform in einer ersten Drehrichtung zu verlagern und das Umkehren der Drehrichtung des Kolbenantriebsmittels den Kolbenkopf (330) hin zur unteren Plattform verlagert.

8. Vorrichtung zum Beproben von Regolith nach Anspruch 1, wobei das Klappenträgerelement (340) innerhalb des mittleren äußeren Gehäuses (302b) verlagerbar ist.

9. Vorrichtung zum Beproben von Regolith nach Anspruch 1, wobei der Schaltradmechanismus (360) ein Schaltradgehäuseelement umfasst, das auf der unteren Plattform getragen wird und ein oberes Schaltradelement (374) aufnimmt, das in Eingriff mit einem unteren Schaltradelement (376) vorgespannt ist, wobei der Schaltradmechanismus vorzugsweise ein Vorspannelement beinhaltet, um das obere Schaltradelement (374) in Wiedereingriff mit dem unteren Schaltradelement (376) zu bringen.

10. Vorrichtung zum Beproben von Regolith nach Anspruch 1, ferner mindestens eine oder mehrere Bilderfassungseinrichtungen (40, 42) beinhaltend, die an der Vorrichtung zum Beproben von Regolith angeordnet sind, um Bilder von deren Betrieb zu empfangen, wobei die Bilderfassungseinrichtungen einen oder mehrere thermoelektrische Kühler (394a, 394b, 395a, 395b) beinhalten, die mit ihnen gekoppelt sind.

11. Vorrichtung zum Beproben von Regolith nach Anspruch 10, wobei eine erste und eine zweite Bilderfassungseinrichtung (40, 42) nahe der Vorrichtung zum Beproben von Regolith angebracht sind, und wobei die erste Bilderfassungseinrichtung (40) und die zweite Bilderfassungseinrichtung (42) mittels eines gemeinsamen Schalters und eines gemeinsamen Datenkabels mit einem gemeinsamen Prozessor gekoppelt sind.

12. Arm zum Beproben von Regolith, eine erste Vorrichtung (30) zum Beproben von Regolith nach einem der Ansprüche 1 bis 11, die an einem ersten Ende angeordnet ist, und eine zweite Vorrichtung (20) zum Beproben von Regolith, die an einem zweiten Ende angeordnet ist, umfassend,
wobei die zweite Vorrichtung (20) zum Beproben von Regolith Folgendes umfasst:
ein Gehäuse,
ein Schaufelelement (210) mit einem Körper zum Aufnehmen von Regolith darin, wobei das Schaufelelement auf das Betätigen eines Schaufelmotors hin, der in dem Gehäuse angeordnet ist, um eine Achse des Gehäuses drehbar ist, und
eine ausfahrbare Zange (220), die dafür konfiguriert ist, auf das Betätigen eines Zangenmotors (230) hin, der mittels eines Schaltradmechanismus, der in dem Gehäuse angeordnet ist, mit der Zange gekoppelt ist, im Wesentlichen Regolith zu umfangen, der in dem Schaufelelement aufgenommen ist.

13. Arm zum Beproben von Regolith nach Anspruch 12, wobei mindestens eines von dem Schaufelelement (210) oder dem ausfahrbaren Zangenelement (220) an seinen Rändern mehrere Kerbverzahnungen beinhaltet.

14. Arm zum Beproben von Regolith nach Anspruch 12, ferner eine oder mehrere Bilderfassungseinrichtungen (40, 42) umfassend, die mittels eines oder mehrerer Wärmeregulierungselemente (394a, 394b, 395a, 395b) mit einem oder mehreren thermoelektrischen Kühlern gekoppelt sind.

15. Arm zum Beproben von Regolith nach Anspruch 12, ferner zwei oder mehr Bilderfassungseinrichtungen (40, 42) umfassend, die über eine Dreizustände-Schalteinrichtung mit einer Steuerung verbunden sind.

## Revendications

1. Appareil d'échantillonnage de régolithe, comprenant :
un boîtier extérieur cylindrique (302) s'étendant autour d'un axe longitudinal comprenant des boîtiers supérieur (302a), intermédiaire (302b) et inférieur (302c) ;
un boîtier intérieur (308) reçu dans ledit boîtier inférieur extérieur et comprenant une paroi circonférentielle définissant un vide (309) à l'intérieur, ledit boîtier intérieur pouvant tourner avec le boîtier extérieur ;
une pluralité de rabats rétractables (304) s'étendant entre le boîtier intérieur (308) et le boîtier extérieur inférieur (302c) ;
un élément de piston comprenant un arbre de piston (370) et une tête de piston (330) disposée dans le boîtier intérieur ;
un élément de support de rabat (340) pouvant être déplacé en translation le long d'une première tige filetée (316) lors de la rotation de celle-ci, ledit élément de support de rabat (340) étant couplé auxdits rabats rétractables (304) pour pousser lesdits rabats dans l'espace entre le boîtier intérieur (308) et le boîtier inférieur (302c) et hors de l'espace entre ceux-ci lors de la translation dudit élément de support de rabat (340) sur ladite tige filetée ;
une plate-forme supérieure (320) isolée de manière rotative et supportée dans le boîtier extérieur supérieur (302a) à une hauteur fixe dans celui-ci, ladite plate-forme supérieure supportant un moyen d'entraînement de rabat (310) couplé de manière rotative à la première tige filetée au moyen d'un premier train d'engrenage (312) et un moyen d'entraînement d'élément de piston (350) couplé au boîtier extérieur pour entraîner de manière rotative ledit boîtier extérieur (302) autour de la plate-forme supérieure (320),
une plate-forme inférieure (380) fixée de manière fixe à la paroi circonférentielle du boîtier intérieur et du boîtier extérieur ; ladite plate-forme inférieure (380) étant couplée de manière rotative au moyen d'un mécanisme à cliquet (360) à une seconde tige filetée comprenant au moins une partie de l'arbre de piston (370) ; ladite seconde tige filetée supportée sur la plate-forme inférieure (380) par un écrou fileté de sorte que la rotation de ladite plate-forme inférieure provoque la translation de la tête de piston (330) à l'intérieur du boîtier inférieur ;
au moins un capteur de détection de limite de position (378) disposé dans le boîtier supérieur (302a) positionné à proximité soit d'une extrémité prédéterminée de déplacement de l'au moins un ou plusieurs éléments parmi la seconde tige filetée destiné à la détection de translation de celle-ci, soit de l'élément de support de rabat (340) ; et
ledit au moins un capteur de détection de position (378) couplé de manière fonctionnelle soit au moyen d'entraînement d'élément de piston (350), soit au moyen d'entraînement de rabat (310).

2. Appareil d'échantillonnage de régolithe selon la revendication 1, dans lequel au moins un capteur de détection de limite de position (378) est disposé dans le boîtier supérieur (302a) positionné à proximité de l'extrémité prédéterminée de déplacement de la seconde tige filetée destiné à la détection de translation de celle-ci ; et au moins un capteur de détection de position (378) est disposé dans le boîtier supérieur positionné à proximité d'une extrémité prédéterminée de déplacement de l'élément de support de rabat.

3. Appareil d'échantillonnage de régolithe selon la revendication 1, dans lequel au moins un capteur de détection de limite de position (378) est couplé de manière fonctionnelle à au moins un parmi le moyen d'entraînement d'élément de piston (350) ou le moyen d'entraînement de rabat (310) par un ou plusieurs microprocesseurs configuré pour interrompre le flux de courant lors d'un actionnement du capteur (378).

4. Appareil d'échantillonnage de régolithe selon la revendication 1, dans lequel au moins un capteur de détection de limite de position (378) disposé dans le boîtier supérieur (302a) est positionné à proximité de l'extrémité prédéterminée de déplacement de la seconde tige filetée destiné à la détection de translation de celle-ci et dans lequel l'au moins un capteur de détection de position est actionné en détectant la proximité d'une saillie (372) sur la seconde tige filetée.

5. Appareil d'échantillonnage de régolithe selon la revendication 1, dans lequel l'au moins un capteur de détection de limite de position (378) disposé dans le boîtier supérieur (302a) est positionné à proximité de l'extrémité prédéterminée de déplacement de l'élément de support de rabat (340) destiné à la détection de translation de celle-ci ; et dans lequel ledit capteur de détection de position est actionné en détectant la proximité d'une saillie sur l'élément de support de rabat.

6. Appareil d'échantillonnage de régolithe selon la revendication 1, dans lequel, si ledit moyen d'entraînement de rabat (310) est configuré pour étendre les rabats (304) depuis l'espace entre le boîtier intérieur et le boîtier extérieur dans une première direction de rotation et l'inversement de ladite direction de rotation dudit moyen d'entraînement des rabat rétracte lesdits rabats.

7. Appareil d'échantillonnage de régolithe selon la revendication 1, dans lequel, si ledit moyen d'entraînement de piston (350) est configuré pour entraîner l'arbre de piston (370), pour provoquer la translation de la tête de piston (330) loin de la plate-forme inférieure dans une première direction de rotation et l'inversement de ladite direction de rotation de moyen d'entraînement de piston provoque la translation de la tête de piston (330) vers la plate-forme inférieure.

8. Appareil d'échantillonnage de régolithe selon la revendication 1, dans lequel l'élément de support de rabat (340) peut être déplacé en translation à l'intérieur du boîtier extérieur central (302b).

9. Appareil d'échantillonnage de régolithe selon la revendication 1, dans lequel le mécanisme à cliquet (360) comprend un élément de boîtier à cliquet supporté sur la plate-forme inférieure et recevant un élément à cliquet supérieur (374) sollicité pour venir en prise avec un élément à cliquet inférieur (376), le mécanisme à cliquet inclut de préférence un élément de sollicitation destiné à pousser l'élément à cliquet supérieur (374) à encore venir en prise avec l'élément à cliquet inférieur (376).

10. Appareil d'échantillonnage de régolithe selon la revendication 1, incluant en outre au moins un ou plusieurs dispositifs d'acquisition d'image (40, 42) disposés sur l'appareil d'échantillonnage de régolithe destinés à recevoir des images du fonctionnement de celui-ci, dans lequel lesdits dispositifs d'acquisition d'image incluent un ou plusieurs refroidisseurs thermoélectriques (394a, 394b, 395a, 395b) couplés à ceux-ci.

11. Appareil d'échantillonnage de régolithe selon la revendication 10, dans lequel des premier et second dispositifs d'acquisition d'image (40, 42) sont fixés à proximité de l'appareil d'échantillonnage de régolithe ; et dans lequel ledit premier dispositif d'acquisition d'image (40) et ledit second dispositif d'acquisition d'image (42) sont couplés au moyen d'un commutateur commun et d'un câble de données commun à un processeur commun.

12. Bras d'échantillonnage de régolithe comprenant un premier appareil d'échantillonnage de régolithe (30) selon l'une quelconque des revendications 1 à 11, disposé à une première extrémité et un second appareil d'échantillonnage de régolithe (20) disposé à une seconde extrémité ;
ledit second appareil d'échantillonnage de régolithe (20) comprenant :
un boîtier ;
un élément de pelle (210) ayant un corps destiné à recevoir du régolithe à l'intérieur de celui-ci ; ledit élément de pelle pouvant tourner autour d'un axe dudit boîtier lors de l'actionnement d'un moteur de pelle disposé dans ledit boîtier ; et
une pince extensible (220) configurée pour enfermer sensiblement le régolithe reçu dans ledit élément de pelle lors de l'actionnement d'un moteur de pince (230) couplé à la pince au moyen d'un mécanisme à cliquet disposé dans ledit boîtier.

13. Bras d'échantillonnage de régolithe selon la revendication 12, dans lequel au moins un parmi l'élément de pelle (210) ou la pince extensible (220) inclut une pluralité de dentelures sur les bords de ceux-ci.

14. Bras d'échantillonnage de régolithe selon la revendication 12, comprenant en outre un ou plusieurs dispositifs d'acquisition d'image (40, 42) reliés au moyen d'un ou plusieurs éléments de régulation thermique (394a, 394b, 395a, 395b) à un ou plusieurs refroidisseurs thermoélectriques.

15. Bras d'échantillonnage de régolithe selon la revendication 12, comprenant en outre deux ou plusieurs dispositifs d'acquisition d'image (40, 42) reliés au moyen d'un dispositif de commutation à trois états avec un dispositif de commande.
